# EUROPÄISCHE PATENTSCHRIFT

(11) **EP 1 674 745 B1**
(45) Veröffentlichungstag und Bekanntmachung des Hinweises auf die Patenterteilung: **10.11.2010**
(21) Anmeldenummer: 05026800.2
(22) Anmeldetag: 08.12.2005
(51) Int. Cl.: F16C 27/04, F16C 27/06, F16C 33/58, F16C 19/06, F16C 19/16, F16C 35/073, A61B 6/03

(54) **Lageranordnung**
Bearing arrangement
Arrangement de palier

(30) Priorität: 23.12.2004 DE 102004062116
(43) Veröffentlichungstag der Anmeldung: 28.06.2006
(73) Patentinhaber: AB SKF, 415 50 Göteborg (SE)
(72) Erfinder: Brandenstein, Manfred, 97776 Eussenheim (DE); Neder, Günter, 97422 Schweinfurt (DE); Neder, Jürgen, 97421 Schweinfurt (DE); Olschewski, Armin, 97422 Schweinfurt (DE); Breunig, Heinz, 63743 Aschaffenburg (DE)
(74) Vertreter: Kohl, Thomas

(56) Entgegenhaltungen:
- DE-A1- 1 575 635
- DE-A1- 2 819 366
- DE-A1- 4 209 320
- DE-B3- 10 333 542
- DE-U- 7 018 297
- US-A1- 2004 062 343

## Beschreibung

Die Erfindung betrifft eine Lageranordnung für ein medizinisches Gerät, mit der ein rotierendes Bauteil relativ zu einem ortsfesten Gehäuse gelagert wird, insbesondere für einen Computertomographen, wobei die Lageranordnung ein Lager mit einem Innenring und einem Außenring aufweist, wobei der Innenring mit dem zu lagernden Bauteil verbunden ist und wobei der Außenring über mindestens ein Dämpfungselement mit dem Gehäuse verbunden ist.

Insbesondere bei Computertomographen besteht die Notwendigkeit, einen trommelförmig ausgebildeten Körper relativ zu einem Gehäuse so zu lagern, dass dieser zwecks Anfertigung von tomographischen Bildern um eine Drehachse gedreht werden kann. In den trommelförmigen Körper ist dabei zwecks Durchstrahlung des zu untersuchenden Patienten eine Röntgenröhre eingebracht sowie auf der diametral zur Röntgenröhre gegenüberliegenden Seite der Trommel Strahlendetektoren, die die emittierte Röntgenstrahlung aufnehmen.

Eine Lösung der gattungsgemäßen Art ist aus der DE-OS 15 75 635 bekannt. Die dort beschriebene Lageranordnung ist für eine elektrische Maschine vorgesehen. Dabei ist der Lageraußenring über ein Dämpfungselement bzw. über mehrere über den Umfang des Lageraußenrings gleichmäßig verteilt angeordnete Dämpfungselemente mit dem Gehäuse bzw. dem Gestell verbunden. Der Lagerinnenring trägt das sich drehende Bauteil.

Eine ebenfalls gattungsgemäße Lageranordnung zeigt die DE 42 09 320 A1. Ähnliche Lösungen zeigen die US 2004/062343 A1, die DE 103 33 542 B3, die DE 70 18 297 U und die DE 28 19 366 A1.

Aus diversen Schriften, beispielsweise aus der DD-PS 78 523, der DE 25 55 021 C2 und der DE 30 32 820 C2, sind ebenfalls Lager für diverse Anwendungen bekannt, bei denen Elastomerblöcke oder aus elastomerem Material bestehende Hülsen eingesetzt werden, um dem Lager eine verbesserte Dämpfungseigenschaft zu verleihen.

Ein Wälzlager für einen Kernspin-Tomographen mit magnetischen Wälzkörpern und mit einem Innenring und einem Außenring, zwischen denen die Wälzkörper abrollen können, ist aus der WO 02/27203 A1 bekannt. Dort ist weiter vorgesehen, dass der Außenring von einem nichtmagnetischen Ring umgeben ist.

Bekannt sind schließlich für computertomographische Anlagen Lageranordnungen, bei denen Draht-Lager mit eingesetzten Dämpfungselementen zur Anwendung kommt. Die drehbare Trommel wird dort also mittels eines Kugellagers mit Laufdrähten gelagert, wobei die Laufdrähte in dämpfenden Kunststoffeinlagen platziert sind. Dabei kommen dann zumeist Keramikkugeln als Wälzkörper für das Lager zum Einsatz.

Es hat sich herausgestellt, dass die vorbekannten Lagersysteme insbesondere in Computertomographen noch nicht den Anforderungen entsprechen, weil die Lagerung im Betrieb, also bei Drehung der Trommel, eine relativ hohe Geräuschentwicklung aufweist. Die Forderung nach einem geräuscharmen Lager, das höchstens 55 dB(A) erzeugt, kann derzeit kaum erfüllt werden. Die Dämpfung der Lageranordnung reicht also bislang nicht aus.

Ein weiteres Problem, das auch in den Zusammenhang mit der Geräuschentwicklung gebracht wird, besteht darin, dass die rotierende Trommel im Betrieb eine nicht unerhebliche Ovalität aufweist, d. h. das Lager ist bislang nicht in der Lage, die Trommel so zu fassen und zu lagern, dass sie eine weitgehend runde Gestalt beibehält.

Der Erfindung liegt daher die **Aufgabe** zugrunde, eine Lageranordnung der eingangs genannten Art so weiterzubilden, dass insbesondere, aber nicht nur, bei Anwendung in einem Computertomographen ein besonders ruhiger und gleichmäßiger Lauf sichergestellt ist. Die Lageranordnung soll insbesondere bei zu lagernden Teilen, die eine relativ geringe Eigensteifigkeit aufweisen, einen guten Rundlauf sicherstellen und so zu erhöhter Laufruhe beitragen.

Die **Lösung** dieser Aufgabe durch die Erfindung ist **dadurch gekennzeichnet, dass** sowohl der Innenring als auch der Außenring als einteilige Elemente ausgebildet sind und eine im wesentlichen hohlzylindrische Grundkontur aufweisen, wobei die Erstreckung des Außenrings in radiale Richtung mindestens das Doppelte der Erstreckung des Innenrings in radiale Richtung beträgt. Vorzugsweise beträgt die Erstreckung des Außenrings in radiale Richtung sogar mindestens das Dreifache derjenigen des Innenrings in radiale Richtung. Ferner ist vorgesehen, dass die Erstreckung des Innenrings in radiale Richtung zwischen 15 mm und 30 mm beträgt, wobei der Innendurchmesser des Innenrings zwischen 1.000 mm und 2.000 mm liegt.

Dabei ist bevorzugt das zu lagernde Bauteil trommelförmig ausgebildet und weist eine im Verhältnis zur Steifigkeit des Außenrings des Lagers geringe Eigensteifigkeit auf, was insbesondere bei der Lagerung der Trommel eines Computertomographen vorteilhaft zur Nutzung kommt.

Das Erfindungskonzept stellt also darauf ab, dass die relativ gering eigensteife Trommel des Tomographen von dem dünnwandigen Innenring des Lagers gefasst wird, wobei durch den im Verhältnis steif ausgebildeten Außenring dem Innenring und in der Folge auch der Trommel eine hohe Rundheit verliehen wird, so dass ein geräuscharmer Lauf der Lageranordnung gewährt wird.

Vorzugsweise ist das Lager als Wälzlager ausgebildet, wobei dann zwischen Innenring und Außenring Wälzkörper, insbesondere Kugeln, angeordnet sind. Dabei sind bevorzugt Innenring, Außenring und dazwischen angeordnete Wälzkörper so toleriert, dass Vorspannung im Lager vorliegt.

Eine Weiterbildung sieht eine Anzahl Dämpfungselemente vor, die äquidistant über den Umfang des Außenrings verteilt angeordnet sind.

Das mindestens eine Dämpfungselement kann in mindestens einer Ausnehmung im Außenring angeordnet sein, wobei das Dämpfungselement und die Ausnehmung so ausgeformt sind, dass das Dämpfungselement im montierten Zustand die hohlzylindrische Grundkontur des Außenrings fortsetzt bzw. ergänzt. Dabei hat die mindestens eine Ausnehmung im Außenring, in axiale Richtung gesehen, vorzugsweise eine bogenförmige, insbesondere kreisbogenförmige, Begrenzung.

Eine optimale Dämpfung bei gleichzeitig hinreichender Steifigkeit wird dadurch erreicht, dass sich gemäß einer Fortbildung das Dämpfungselement von der Anlagefläche am Gehäuse in axiale Richtung zwischen 30 % und 70 % der axialen Breite des Außenrings erstreckt.

Eine optimale Anbindung des Außenrings am Gehäuse wird dadurch erreicht, dass weiterbildungsgemäß die Erstreckung des Dämpfungselements in axiale Richtung größer ist als die axiale Breite der Ausnehmung im Außenring zur Aufnahme des Dämpfungselements. Dabei hat es sich bewährt, wenn die Erstreckung des Dämpfungselements in axiale Richtung zwischen 102 % und 107 % der axialen Breite der Ausnehmung im Außenring beträgt.

Als weiterbildende Ausgestaltung kann auch vorgesehen werden, dass Dämpfungselemente in beiden axialen Endbereichen des Außenrings in Ausnehmungen angeordnet sind. Der Außenring wird dann also beiderseits von Dämpfungselementen begrenzt und über diese am Gehäuse angebunden.

Zur Festlegung des Außenrings am Gehäuse ist bevorzugt vorgesehen, dass sich Befestigungsmittel, insbesondere Schrauben, in axiale Richtung durch den Außenring, das Dämpfungselement bzw. die Dämpfungselemente und das Gehäuse erstrecken.

Ein Schutz der Dämpfungselemente von außen kann erreicht werden, wenn das Dämpfungselement bzw. die Dämpfungselemente von einem Abdeckteil abgedeckt wird bzw. werden, das sich über einen Teil des Außenumfangs des Außenrings erstreckt und am Außenring festgelegt, insbesondere festgeschraubt, ist.

Das Vorhandensein einer hinreichenden Vorspannung im Lager begünstigt die Zentrierwirkung, die der relativ massiv ausgebildete Außenring des Lagers über die Wälzkörper und den Innenring auf die relativ wenig eigensteife Trommel ausübt. Daher sind mit Vorteil Mittel vorgesehen, mit denen die Vorspannung im Lager eingestellt werden kann. Diese Mittel können durch eine konische Hülse gebildet werden, die an einem der Lagerringe anliegt und die in axiale Richtung einstellbar verschiebbar angeordnet ist. Vorzugsweise wirkt die konische Hülse mit dem Innenring zusammen; besonders bevorzugt ist sie zwischen Bauteil und Innenring angeordnet. Eine vereinfache Montagemöglichkeit ergibt sich, wenn gemäß einer weiteren Fortbildung vorgesehen wird, dass die konische Hülse durch einzelne Segmente, insbesondere durch vier Segmente, gebildet wird, die in Umfangsrichtung aneinandergrenzend angeordnet sind.

Die konische Hülse kann aus Kunststoff bestehen. Als Material für das Dämpfungselement hat sich Gummi oder Elastomermaterial, insbesondere thermoplastischer oder duroplastischer Kunststoff, bewährt. Während die Lagerringe aus üblichem Kugellagerstahl bestehen können, ist es auch möglich, dass sie aus nicht-magnetischem Material bestehen.

Mit der erfindungsgemäßen Ausgestaltung wird eine Lageranordnung geschaffen, die sich durch einen besonders ruhigen Lauf auszeichnet. Bei der Anwendung in Computertomographen werden Geräuschentwicklung durch die Lagerung von weniger als 55 dB(A) möglich.

Die zu lagernde Trommel des Tomographen wird durch die vorgeschlagene Lageranordnung gut zentriert gehalten, so dass die Ovalität der Trommel gering ist.

Die auf das System wirkenden Schwingungen werden - sowohl in axialer als auch in radialer Richtung - gut gedämpft.

In der Zeichnung sind Ausführungsbeispiele der Erfindung dargestellt. Es zeigen:
Figur 1 den Schnitt durch eine Lageranordnung (Schnitt X-X gemäß Figur 2 bzw. 3),
Figur 2 den Lageraußenring samt Dämpfungselement gemäß Schnitt Y-Y von Fig. 1,
Figur 3 in perspektivischer Ansicht einen Teil der Lageranordnung mit teilweise geschnittenen Teilen und
Figur 4 eine zu Fig. 1 alternative Ausführungsform des Außenrings samt Dämpfungselementen.

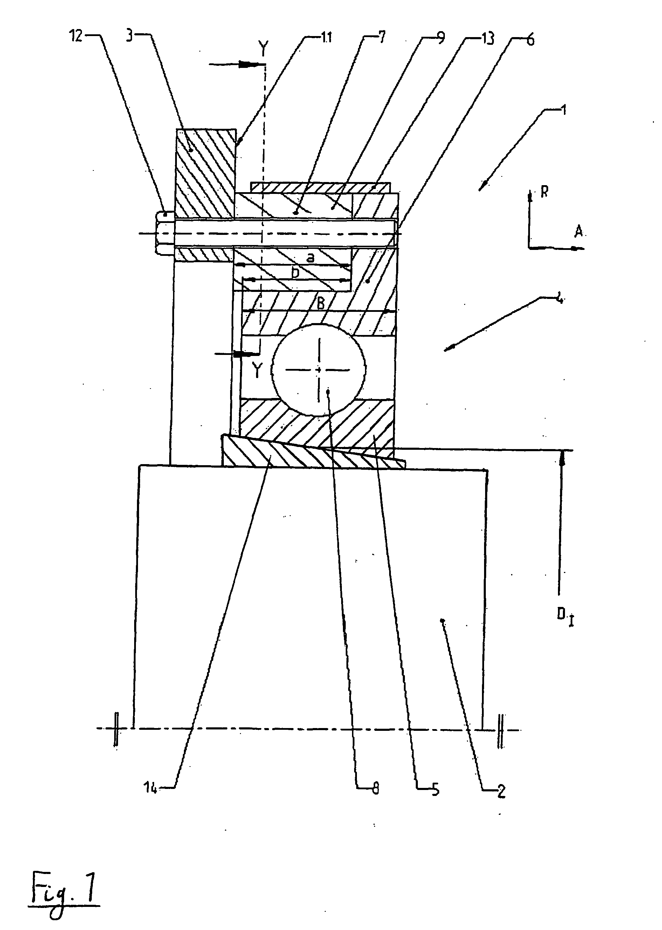

In Figur 1 ist der Schnitt durch die obere Hälfte einer Lageranordnung 1 zu sehen. Die Lageranordnung 1 lagert ein rotierendes Bauteil 2 in Form der Trommel eines Computertomographen relativ zu einem ortsfesten Gehäuse 3. Das Bauteil 2 ist dabei aus Gewichtsgründen relativ dünnwandig ausgebildet, so dass es leicht zu einer unerwünschten Ovalität neigt.

Die Lagerung des Bauteils 2 wird durch ein Lager 4 bewerkstelligt, das vorliegend als einreihiges Rillenkugellager ausgebildet ist. Es ist aber genauso möglich, andere Lagertypen einzusetzen. Das Lager 4 weist einen Innenring 5 und einen Außenring 6 auf, zwischen denen in bekannter Weise Wälzkörper 8 in Form von Kugeln angeordnet sind. Je nach Bedarf bestehen die Kugeln 8 entweder klassisch aus Stahl oder aus Keramik.

Wie der Schnittdarstellung von Figur 1 gut entnommen werden kann, sind sowohl der Innenring 5 als auch der Außenring 6 als einteilige Elemente ausgebildet, d. h. sie bestehen aus einem einstückigen Ring. Ferner weisen beide Ringe 5, 6 eine im wesentlichen hohlzylindrische Grundkontur auf. Der Innenring 5 ist im Verhältnis zum Außenring 6 relativ dünnwandig ausgebildet. Namentlich ist die radiale Erstreckung des Außenrings 6 (s. radiale Richtung R) mindestens doppelt so groß, vorzugsweise drei Mal so groß, wie die radiale Erstreckung des Innenrings 5. Dabei ist das gesamte Lager 4 als sog. Großlager ausgebildet, d. h. der Außendurchmesser des Außenrings 6 ist größer als ca. 400 mm.

Im Ausführungsbeispiel beträgt die radiale Erstreckung des Innenrings 5 ca. 15 mm bis 30 mm, wobei der Innendurchmesser D_{I} des Innenrings 5 zwischen 1.000 mm und 2.000 mm liegt.

Wie in der Zusammenschau der Figuren 1 bis 3 zu sehen ist, weist der Außenring 6 an einigen Umfangsstellen, beispielsweise an vier, fünf oder sechs Stellen des Umfangs, eine Ausnehmung 9 auf, in die ein Dämpfungselement 7 eingesetzt ist. Das Dämpfungselement 7 stellt die Verbindung zwischen Außenring 6 und Gehäuse 3 her, wozu Schrauben 12 zum Einsatz kommen, die, in Achsrichtung A ausgerichtet, Gehäuse 3, Dämpfungselement 7 und Außenring 6 durchsetzen und verspannen, was in den Figuren nur schematisch angedeutet ist.

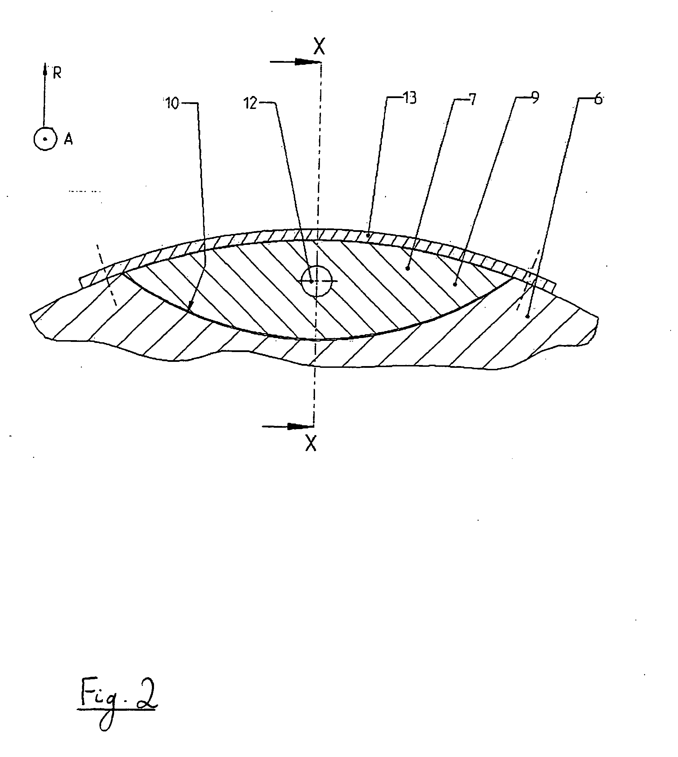

Wie am besten aus Figur 2 zu sehen ist, hat die Ausnehmung 9 für das Dämpfungselement 7 eine bogenförmige Begrenzung 10. Das Dämpfungselement 7 ist wiederum so ausgeformt, dass es nach seinem Einsetzen in die Ausnehmung 9 diese derart füllt, dass sich eine weitgehend ungestörte hohlzylindrische Kontur für den Außenring 6 ergibt.

Aus Figur 1 ist zu entnehmen, dass die Ausnehmung 9 im Außenring 6 in Achsrichtung A eine Breite b hat, die etwa zwei Drittel der axialen Breite B des Außenrings 6 entspricht. Das einzusetzende Dämpfungselement 7 hat indes - zumindest im nicht montierten und spannungsfreien Zustand ― eine axiale Erstreckung a, die geringfügig größer ist als die Breite b, so dass das Dämpfungselement 7 geringfügig nach links über die Seite des Außenrings 6 übersteht. Der überstehende Teil des Dämpfungselements 7 liegt an der Anlagefläche 11 am Gehäuse 3 an, so dass der Kontakt zwischen Gehäuse 3 und Außenring 6 insoweit gänzlich über das Dämpfungselement 7 hergestellt werden kann. Bei entsprechendem Anziehen der Schrauben 12 kann freilich infolge der Kompression des Dämpfungselements 7 eine Anlage der Seite des Außenrings 6 an der Anlagefläche 11 am Gehäuse 3 erreicht werden.

Das Dämpfungselement 7 kann - wie es aus den Figuren 1 und 2 hervorgeht - durch ein Abdeckteil 13 abgedeckt werden. Das Abdeckteil 13 wird am Außenumfang des Außenrings 6 festgeschraubt; es erstreckt sich über einen gewissen Umfangsabschnitt des Außenrings 6.

Die optimale Lagerung der relativ wenig eigenstabilen Trommel 2 durch die Lageranordnung 1 ergibt sich durch den gleichermaßen gering eigensteifen Innenring 5 des Lagers 4, der mit dem im Verhältnis dazu sehr massiv ausgebildeten, stabilen Außenring 6 zusammenwirkt. Über eine Vorspannung des Lagers 4, d. h. über eine Pressung zwischen Außenring 6, Kugeln 8 und Innenring 5, wird zunächst der Innenring 5 und in der Folge auch die Trommel 2 rund gedrückt, so dass die Ovalität der Trommel im gelagerten Zustand gering ist.

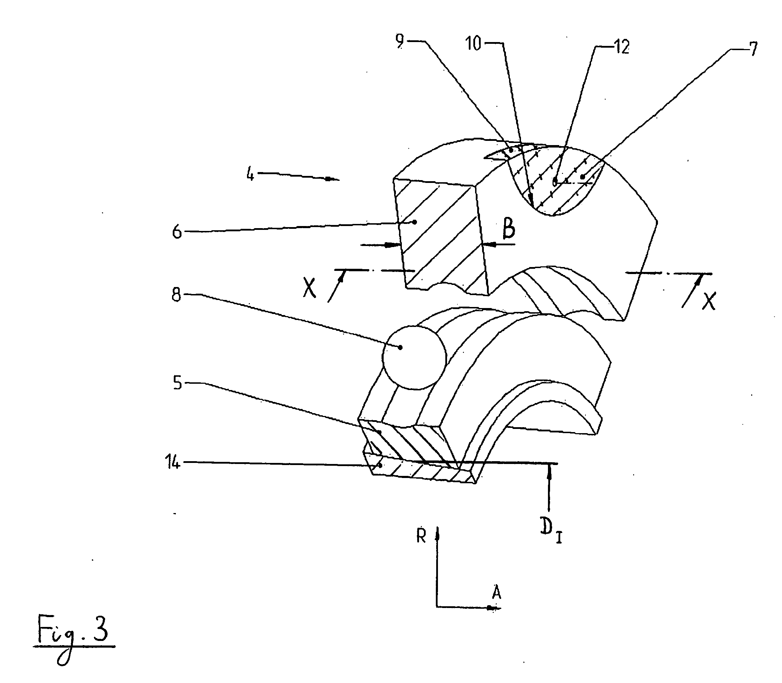

Damit im Lager 4 eine hinreichende Vorspannung vorhanden ist, sind Mittel 14 zur Einstellung der Vorspannung vorhanden, wobei es sich bei diesen Mitteln 14 um eine konische Hülse handelt, die mit dem an seiner radial innenliegenden Fläche des Innenrings 5 ebenfalls konisch ausgebildeten Fläche zusammenwirkt, wie es aus Figur 1 und Figur 3 zu sehen ist. Durch axiale Verstellung der konischen Hülse 14 relativ zum Innenring 5 kann das gewünschte Maß an Vorspannung im Lager 4 in an sich bekannter Weise eingestellt werden.

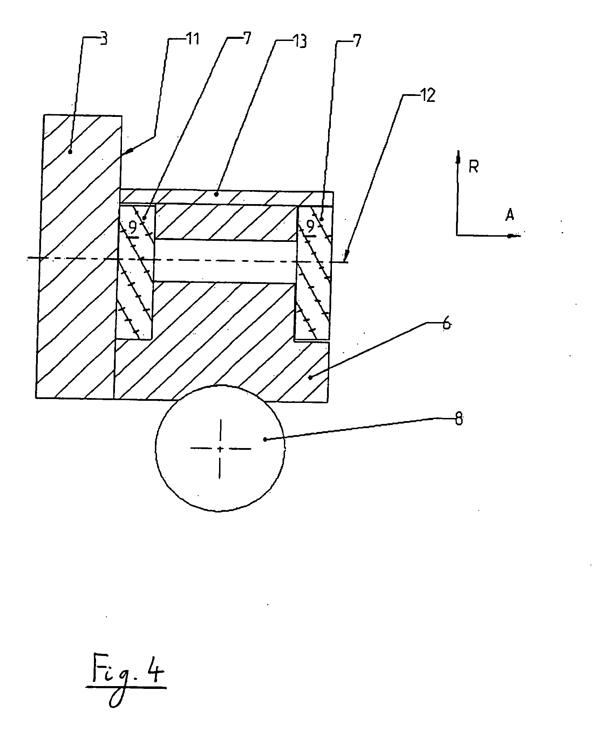

In Figur 4 ist eine alternative Ausführungsform zu sehen. Hier ist vorgesehen, dass der Außenring 6 in beiden axialen Endbereichen mit einer Ausnehmung 9 versehen ist, in die jeweils Dämpfungselemente 7 eingesetzt sind. Auch hier sind über den Umfang des Außenrings 6 mehrere dieser Ausnehmungen 9 bzw. Dämpfungselemente 7 angeordnet. Mit dieser Lösung wird eine noch weichere bzw. gedämpftere Anbindung des Außenrings 6 am Gehäuse 3 möglich.

Um ein Optimum an Geräuschreduzierung zu erreichen, können die WälzkörperLaufbahnen in Außen- und Innenring nach dem Schleifen gehont oder rolliert werden. D. h. nach dem Schleifen wird eine weitere Feinbearbeitung vorgesehen, um bestmögliche Resultate zu erzielen.

### Bezugszeichenliste

- 1: Lageranordnung
- 2: rotierendes Bauteil
- 3: ortsfestes Gehäuse
- 4: Lager
- 5: Innenring
- 6: Außenring
- 7: Dämpfungselement
- 8: Wälzkörper (Kugeln)
- 9: Ausnehmung
- 10: bogenförmige Begrenzung
- 11: Anlagefläche am Gehäuse
- 12: Befestigungsmittel (Schraube)
- 13: Abdeckteil
- 14: Mittel zur Einstellung der Vorspannung (konische Hülse)

- R: radiale Richtung
- A: axiale Richtung
- D_{I}: Innendurchmesser
- B: axiale Breite des Außenrings
- a: Erstreckung des Dämpfungselements in axiale Richtung
- b: axiale Breite der Ausnehmung

## Patentansprüche

1. Lageranordnung (1) für ein medizinisches Gerät, mit der ein rotierendes Bauteil (2) relativ zu einem ortsfesten Gehäuse (3) gelagert wird, insbesondere für einen Computertomographen, wobei die Lageranordnung (1) ein Lager (4) mit einem Innenring (5) und einem Außenring (6) aufweist, wobei der Innenring (5) mit dem zu lagernden Bauteil (2) verbunden ist und wobei der Außenring (6) über mindestens ein Dämpfungselement (7) mit dem Gehäuse (3) verbunden ist, **dadurch gekennzeichnet, dass** sowohl der Innenring (5) als auch der Außenring (6) als einteilige Elemente ausgebildet sind und eine im wesentlichen hohlzylindrische Grundkontur aufweisen, wobei die Erstreckung des Außenrings (6) in radiale Richtung (R) mindestens das Doppelte der Erstreckung des Innenrings (5) in radiale Richtung (R) beträgt, wobei die Erstreckung des Innenrings (5) in radiale Richtung (R) zwischen 15 mm und 30 mm beträgt und wobei der Innendurchmesser (D₁) des Innenrings (5) zwischen 1.000 mm und 2.000 mm liegt.

2. Lageranordnung nach Anspruch 1, **dadurch gekennzeichnet, dass** die Erstreckung des Außenrings (6) in radiale Richtung (R) mindestens das Dreifache der Erstreckung des Innenrings (6) in radiale Richtung (R) beträgt.

3. Lageranordnung nach Anspruch 1 oder 2, **dadurch gekennzeichnet, dass** das zu lagernde Bauteil (2) trommelförmig ausgebildet ist und eine im Verhältnis zur Steifigkeit des Außenrings (6) des Lagers (4) geringe Eigensteifigkeit aufweist.

4. Lageranordnung nach einem der Ansprüche 1 bis 3, **dadurch gekennzeichnet, dass** das Lager (4) als Wälzlager ausgebildet ist und zwischen Innenring (5) und Außenring (6) Wälzkörper (8), insbesondere Kugeln, angeordnet sind.

5. Lageranordnung nach Anspruch 4, **dadurch gekennzeichnet, dass** Innenring (5), Außenring (6) und dazwischen angeordnete Wälzkörper (8) so toleriert sind, dass Vorspannung im Lager (4) vorliegt.

6. Lageranordnung nach einem der Ansprüche 1 bis 5, **gekennzeichnet durch** eine Anzahl Dämpfungselemente (7), die äquidistant über den Umfang des Außenrings (6) verteilt angeordnet sind.

7. Lageranordnung nach einem der Ansprüche 1 bis 6, **dadurch gekennzeichnet, dass** das mindestens eine Dämpfungselement (7) in mindestens einer Ausnehmung (9) im Außenring (6) angeordnet ist, wobei das Dämpfungselement (7) und die Ausnehmung (9) so ausgeformt sind, dass das Dämpfungselement (7) im montierten Zustand die hohlzylindrische Grundkontur des Außenrings (6) fortsetzt bzw. ergänzt.

8. Lageranordnung nach Anspruch 7, **dadurch gekennzeichnet, dass** die mindestens eine Ausnehmung (9) im Außenring (6), in axiale Richtung (A) gesehen, eine bogenförmige, insbesondere kreisbogenförmige, Begrenzung (10) aufweist.

9. Lageranordnung nach Anspruch 7 oder 8, **dadurch gekennzeichnet, dass** sich das Dämpfungselement (7) von der Anlagefläche (11) am Gehäuse (3) in axiale Richtung (A) zwischen 30 % und 70 % der axialen Breite (B) des Außenrings (6) erstreckt.

10. Lageranordnung nach einem der Ansprüche 7 bis 9, **dadurch gekennzeichnet, dass** die Erstreckung (a) des Dämpfungselements (7) in axiale Richtung (A) größer ist als die axiale Breite (b) der Ausnehmung (9) im Außenring (3) zur Aufnahme des Dämpfungselements (7).

11. Lageranordnung nach Anspruch 10, **dadurch gekennzeichnet, dass** die Erstreckung (a) des Dämpfungselements (7) in axiale Richtung (A) zwischen 102 % und 107 % der axialen Breite (b) der Ausnehmung (9) im Außenring (3) beträgt.

12. Lageranordnung nach einem der Ansprüche 7 bis 11, **dadurch gekennzeichnet, dass** Dämpfungselemente (7) in beiden axialen Endbereichen des Außenrings (6) in Ausnehmungen (9) angeordnet sind.

13. Lageranordnung nach einem der Ansprüche 7 bis 12, **dadurch gekennzeichnet, dass** sich Befestigungsmittel (12), insbesondere Schrauben, zum Festlegen des Außenrings (6) am Gehäuse (3) in axiale Richtung (A) durch den Außenring (6), das Dämpfungselement bzw. die Dämpfungselemente (7) und das Gehäuse (3) erstrecken.

14. Lageranordnung nach einem der Ansprüche 1 bis 13, **dadurch gekennzeichnet, dass** das Dämpfungselement bzw. die Dämpfungselemente (7) von einem Abdeckteil (13) abgedeckt wird bzw. werden, das sich über einen Teil des Außenumfangs des Außenrings (6) erstreckt und am Außenring (6) festgelegt, insbesondere festgeschraubt, ist.

15. Lageranordnung nach einem der Ansprüche 1 bis 14, **gekennzeichnet durch** Mittel (14), mit denen die Vorspannung im Lager (4) eingestellt werden kann.

16. Lageranordnung nach Anspruch 15, **dadurch gekennzeichnet, dass** das Mittel (14) eine konische Hülse ist, die an einem der Lagerringe (5, 6) anliegt und die in axiale Richtung (A) einstellbar verschiebbar angeordnet ist.

17. Lageranordnung nach Anspruch 16, **dadurch gekennzeichnet, dass** die konische Hülse (14) mit dem Innenring (5) zusammenwirkt.

18. Lageranordnung nach Anspruch 17, **dadurch gekennzeichnet, dass** die konische Hülse (13) zwischen Bauteil (2) und Innenring (5) angeordnet ist.

19. Lageranordnung nach einem der Ansprüche 16 bis 18, **dadurch gekennzeichnet, dass** die konische Hülse (14) aus Kunststoff besteht.

20. Lageranordnung nach einem der Ansprüche 16 bis 19, **dadurch gekennzeichnet, dass** die konische Hülse (14) durch einzelne Segmente, insbesondere durch vier Segmente, gebildet wird, die in Umfangsrichtung aneinandergrenzend angeordnet sind.

21. Lageranordnung nach einem der Ansprüche 1 bis 20, **dadurch gekennzeichnet, dass** das Dämpfungselement (7) aus Gummi oder aus Elastomermaterial, insbesondere aus thermoplastischem oder aus duroplastischem Kunststoff, besteht.

22. Lageranordnung nach einem der Ansprüche 1 bis 21, **dadurch gekennzeichnet, dass** die Lagerringe (5, 6) aus nicht-magnetischem Material bestehen.

## Claims

1. Bearing arrangement (1) for a medical instrument, by means of which a rotating component (2) is borne relative to a fixed housing (3), more particularly for a computed tomography scanner, the bearing arrangement (1) having a bearing (4) with an inner race (5) and an outer race (6), the inner race (5) being connected to the component (2) to be borne and the outer race (6) being connected to the housing (3) via at least one damping element (7), **characterized in that** both the inner race (5) and the outer race (6) are designed as integral elements and have a substantially hollow-cylindrical basic contour, the extent of the outer race (6) in the radial direction (R) being at least two times the extent of the inner race (5) in the radial direction (R), with the extent of the inner race (5) in the radial direction (R) being between 15 mm and 30 mm, and the inner diameter (D₁) of the inner race (5) lying between 1000 mm and 2000 mm.

2. Bearing arrangement according to Claim 1, **characterized in that** the extent of the outer race (6) in the radial direction (R) is at least three times the extent of the inner race (5) in the radial direction (R).

3. Bearing arrangement according to Claim 1 or 2, **characterized in that** the component (2) to be borne has a drum-like design and has low inherent stiffness compared to the stiffness of the outer race (6) of the bearing (4).

4. Bearing arrangement according to one of Claims 1 to 3, **characterized in that** the bearing (4) is designed as a rolling-element bearing and roll bodies (8), more particularly spheres, are arranged between inner race (5) and outer race (6).

5. Bearing arrangement according to Claim 4, **characterized in that** inner race (5), outer race (6) and roll bodies (8) arranged therebetween have such tolerances that there is pretension in the bearing (4).

6. Bearing arrangement according to one of Claims 1 to 5, **characterized by** a number of damping elements (7) that are arranged distributed equidistantly over the circumference of the outer race (6).

7. Bearing arrangement according to one of Claims 1 to 6, **characterized in that** the at least one damping element (7) is arranged in at least one cutout (9) in the outer race (6), the damping element (7) and the cutout (9) being shaped such that the damping element (7), in the mounted state, continues or complements the hollow-cylindrical basic contour of the outer race (6).

8. Bearing arrangement according to Claim 7, **characterized in that** the at least one cutout (9) in the outer race (6), as seen in the axial direction (A), has an arc-shaped, more particularly circular-arc-shaped, boundary (10).

9. Bearing arrangement according to Claim 7 or 8, **characterized in that**, in the axial direction (A), the damping element (7) extends from the contact surface (11) on the housing (3) to between 30% and 70% of the axial width (B) of the outer race (6).

10. Bearing arrangement according to one of Claims 7 to 9, **characterized in that** the extent (a) of the damping element (7) in the axial direction (A) is greater than the axial width (b) of the cutout (9), in the outer race (6), for holding the damping element (7).

11. Bearing arrangement according to Claim 10, **characterized in that** the extent (a) of the damping element (7) in the axial direction (A) lies between 102% and 107% of the axial width (b) of the cutout (9) in the outer race (6).

12. Bearing arrangement according to one of Claims 7 to 11, **characterized in that** damping elements (7) are arranged in cutouts (9) in both axial end regions of the outer race (6).

13. Bearing arrangement according to one of Claims 7 to 12, **characterized in that** attachment means (12), more particularly screws, for fixing the outer race (6) on the housing (3) extend through the outer race (6), the damping element or the damping elements (7) and the housing (3) in the axial direction (A).

14. Bearing arrangement according to one of Claims 1 to 13, **characterized in that** the damping element or the damping elements (7) is or are covered by a cover part (13), which extends over part of the outer circumference of the outer race (6) and is fixed, more particularly screwed tight, onto the outer race (6).

15. Bearing arrangement according to one of Claims 1 to 14, **characterized by** means (14) that can be used to set the pretension in the bearing (4).

16. Bearing arrangement according to Claim 15, **characterized in that** the means (14) is a conical sleeve, which rests against one of the bearing races (5, 6) and is arranged such that it can be displaced in the axial direction (A) in an adjustable fashion.

17. Bearing arrangement according to Claim 16, **characterized in that** the conical sleeve (14) interacts with the inner race (5).

18. Bearing arrangement according to Claim 17, **characterized in that** the conical sleeve (14) is arranged between component (2) and inner race (5).

19. Bearing arrangement according to one of Claims 16 to 18, **characterized in that** the conical sleeve (14) consists of plastic.

20. Bearing arrangement according to one of Claims 16 to 19, **characterized in that** the conical sleeve (14) is formed by individual segments, more particularly by four segments, which are arranged adjacent to one another in the circumferential direction.

21. Bearing arrangement according to one of Claims 1 to 20, **characterized in that** the damping element (7) consists of rubber or an elastomeric material, more particularly a thermoplastic or thermosetting plastic.

22. Bearing arrangement according to one of Claims 1 to 21, **characterized in that** the bearing races (5, 6) consist of a nonmagnetic material.

## Revendications

1. Agencement de palier (1) pour un appareil médical, qui permet de supporter un composant rotatif (2) par rapport à un boîtier fixe (3), notamment pour un tomodensitomètre, dans lequel l'agencement de palier (1) présente un palier (4) avec une bague interne (5) et une bague externe (6), la bague interne (5) étant connectée au composant (2) à supporter, et la bague externe (6) étant connectée au boîtier (3) par le biais d'au moins un élément d'amortissement (7), **caractérisé en ce que** la bague interne (5) ainsi que la bague externe (6) sont réalisées sous forme d'éléments d'une seule pièce et présentent un contour de base essentiellement cylindrique creux, l'étendue de la bague externe (6) dans la direction radiale (R) représentant au moins le double de l'étendue de la bague interne (5) dans la direction radiale (R), l'étendue de la bague interne (5) dans la direction radiale (R) étant comprise entre 15 mm et 30 mm et le diamètre intérieur (D_{I}) de la bague interne. (5) étant compris entre 1 000 mm et 2 000 mm.

2. Agencement de palier selon la revendication 1, **caractérisé en ce que** l'étendue de la bague externe (6) dans la direction radiale (R) représente au moins le triple de l'étendue de la bague interne (5) dans la direction radiale (R).

3. Agencement de palier selon la revendication 1 ou 2, **caractérisé en ce que** le composant (2) à supporter est réalisé sous forme de tambour et présente une rigidité propre faible par rapport à la rigidité de la bague externe (6) du palier (4).

4. Agencement de palier selon l'une quelconque des revendications 1 à 3, **caractérisé en ce que** le palier (4) est réalisé sous forme de palier à roulement et des corps de roulement (8), en particulier des billes, sont disposés entre la bague interne (5) et la bague externe (6).

5. Agencement de palier selon la revendication 4, **caractérisé en ce que** la bague interne (5), la bague externe (6) et les corps de roulement (8) disposés entre elles sont pourvus de tolérances telles qu'il existe une précontrainte dans le palier (4).

6. Agencement de palier selon l'une quelconque des revendications 1 à 5, **caractérisé par** une pluralité d'éléments d'amortissement (7), qui sont répartis de manière équidistante sur la périphérie de la bague externe (6).

7. Agencement de palier selon l'une quelconque des revendications 1 à 6, **caractérisé en ce que** l'au moins un élément d'amortissement (7) est disposé dans au moins un évidement (9) dans la bague externe (6), l'élément d'amortissement (7) et l'évidement (9) étant formés de telle sorte que l'élément d'amortissement (7) continue ou complète le contour de base cylindrique creux de la bague externe (6) dans l'état monté.

8. Agencement de palier selon la revendication 7, **caractérisé en ce que** l'au moins un évidement (9) dans la bague externe (6), vu dans la direction axiale (A), présente une limitation (10) de forme courbe, notamment en forme d'arc de cercle.

9. Agencement de palier selon la revendication 7 ou 8, **caractérisé en ce que** l'élément d'amortissement (7) s'étend depuis la surface d'appui (11) sur le boîtier (3) dans la direction axiale (A) sur entre 30% et 70% de la largeur axiale (B) de la bague externe (6).

10. Agencement de palier selon l'une quelconque des revendications 7 à 9, **caractérisé en ce que** l'étendue (a) de l'élément d'amortissement (7) dans la direction axiale (A) est plus grande que la largeur axiale (b) de l'évidement (9) dans la bague externe (6) pour recevoir l'élément d'amortissement (7).

11. Agencement de palier selon la revendication 10, **caractérisé en ce que** l'étendue (a) de l'élément d'amortissement (7) dans la direction axiale (A) représente entre 102% et 107% de la largeur axiale (b) de l'évidement (9) dans la bague externe (6).

12. Agencement de palier selon l'une quelconque des revendications 7 à 11, **caractérisé en ce que** des éléments d'amortissement (7) sont disposés dans les deux régions d'extrémité axiales de la bague externe (6) dans des évidements (9).

13. Agencement de palier selon l'une quelconque des revendications 7 à 12, **caractérisé en ce que** des moyens de fixation (12), en particulier des vis pour la fixation de la bague externe (6) sur le boîtier (3), s'étendent dans la direction axiale (A) à travers la bague externe (6), l'élément d'amortissement ou les éléments d'amortissement (7) et le boîtier (3).

14. Agencement de palier selon l'une quelconque des revendications 1 à 13, **caractérisé en ce que** l'élément d'amortissement ou les éléments d'amortissement (7) est ou sont recouvert(s) par une partie de recouvrement (13), qui s'étend sur une partie de la périphérie extérieure de la bague externe (6), et qui est fixée sur la bague externe (6), notamment par vissage.

15. Agencement de palier selon l'une quelconque des revendications 1 à 14, **caractérisé par** des moyens (14) avec lesquels la précontrainte dans le palier (4) peut être ajustée.

16. Agencement de palier selon la revendication 15, **caractérisé en ce que** le moyen (14) est une douille conique qui s'applique contre l'une des bagues de palier (5, 6), et qui est disposée de manière déplaçable et ajustable dans la direction axiale (A).

17. Agencement de palier selon la revendication 16, **caractérisé en ce que** la douille conique (14) coopère avec la bague interne (5).

18. Agencement de palier selon la revendication 17, **caractérisé en ce que** la douille conique (14) est disposée entre le composant (2) et la bague interne (5).

19. Agencement de palier selon l'une quelconque des revendications 16 à 18, **caractérisé en ce que** la douille conique (14) se compose de plastique.

20. Agencement de palier selon l'une quelconque des revendications 16 à 19, **caractérisé en ce que** la douille conique (14) est formée par des segments individuels, notamment par quatre segments, qui sont disposés les uns à côté des autres dans la direction périphérique.

21. Agencement de palier selon l'une quelconque des revendications 1 à 20, **caractérisé en ce que** l'élément d'amortissement (7) se compose de caoutchouc ou de matériau élastomère, notamment de plastique thermoplastique ou de plastique duroplastique.

22. Agencement de palier selon l'une quelconque des revendications 1 à 21, **caractérisé en ce que** les bagues de palier (5, 6) se composent de matériau non magnétique.
